# EUROPÄISCHE PATENTSCHRIFT

(11) **EP 0 428 166 B1**
(45) Veröffentlichungstag und Bekanntmachung des Hinweises auf die Patenterteilung: **06.07.1994**
(21) Anmeldenummer: 90121857.8
(22) Anmeldetag: 15.11.1990
(51) Int. Cl.: C07C 17/16, C07C 19/02

(54) **Verfahren zur Herstellung von Methylhalogeniden**
Process for preparing methylhalides
Procédé pour la préparation d'halogénures de méthyle

(30) Priorität: 16.11.1989 DE 3938089
(43) Veröffentlichungstag der Anmeldung: 22.05.1991
(73) Patentinhaber: WACKER-CHEMIE GMBH, D-81737 München (DE)
(72) Erfinder: Winkler, Peter-Paul, Dr. Chem., W-8261 Kastl (DE); Goetze, Ulrich, Dr. Chem., W-8623 Burghausen (DE)

(56) Entgegenhaltungen:
- US-A- 3 852 368
- US-A- 3 981 938
- SOVIET INVENTIONS ILLUSTRATED, CH Sektion, Woche C 34, 1. Oktober 1980, DERWENT PUBLICATIONS LTD., London, C 03
- SOVIET INVENTIONS ILLUSTRATED, CH Sektion, Woche C 33, 24. September 1980, DERWENT PUBLICATIONS LTD., London, C 01
- PATENT ABSTRACTS OF JAPAN, unexamined applications, C Sektion, Band 3, Nr. 50, 27. April 1979, THE PATENT OFFICE JAPANESE GOVERNMENT, Seite 20 C 44
- PATENT ABSTRACTS OF JAPAN, unexamined applications, C Sektion, Band 6, Nr. 31, 24. Februar 1982, THE PATENT OFFICE JAPANESE GOVERNMENT, Seite 94 C 92
- PATENT ABSTRACTS OF JAPAN, unexamined applications, C Sektion, Band 7, Nr. 219, 29. September 1983, THE PATENT OFFICE JAPANESE GOVERNMENT, Seite 165 C 188

## Beschreibung

Die Erfindung betrifft ein Verfahren zur Herstellung von Methylhalogeniden in Gegenwart von Aminhydrohalogenid.

Verfahren zur Herstellung von Halogenalkanen sind vielfach bekannt. Dabei handelt es sich im wesentlichen um katalysierte und nichtkatalysierte Verfahren in Gasphase oder Flüssigphase.

In US-PS 3,981,938 (J.M. Steele, Dow Chemical Co., ausgegeben am 21. September 1976) wird beispielsweise die Herstellung von Halogenalkanen in flüssiger Phase durch Umsetzung von Alkanol mit einem mindestens 10 %igen Überschuß an Halogenwasserstoff beschrieben, wobei das anfallende Reaktionswasser am Sumpf des Flüssigphasenreaktors im Gemisch mit Alkanol und Halogenwasserstoff abgezogen wird. Ein ähnliches Verfahren ist aus US-PS 3,983,180 (Shinetsu Chemical Co., ausgegeben am 28. September 1976) bekannt, bei dem in flüssiger Phase Methanol mit Chlorwasserstoff zu Chlormethan umgesetzt wird, wobei die Konzentration des Chlorwasserstoffs über der Azeotropkonzentration liegt. Beide Verfahren haben den Nachteil, daß die Aufarbeitung der ausgeschleusten wäßrigen Lösung von Halogenwasserstoff kostenintensiv und mit einem hohen apparativen Aufwand verbunden ist. In US-PS 4,366,324 (Shinetsu Chemical Co., ausgegeben am 28. Dezember 1982) wird ein Verfahren zur gleichzeitigen Herstellung von Organosiloxanen und Chlormethan aus Organochlorsilan und Methanol in flüssiger Phase beschrieben, wobei die Konzentration des Chlorwasserstoffs in der flüssigen Phase unter der Azeotropkonzentration liegt. Die Nachteile dieses Verfahrens liegen hauptsächlich in der vergleichsweise niedrigen Raum-Zeit-Ausbeute und in dem relativ hohen Anteil an Nebenprodukt Dimethylether, was die Reinigung von Chlormethan zusätzlich erschwert.

In US-PS 4,108,882 (L.G. Mahone, Dow Corning Co., ausgegeben am 22. August 1978) ist ein Verfahren zur gleichzeitigen Herstellung von Siloxanen und Chlormethan in der Gasphase offenbart, bei dem Organochlorsilan und Methanol in Anwesenheit von quarternärer Ammoniumverbindung als Katalysator umgesetzt wird, wobei Temperaturen und Druck so gewählt werden, daß das entstehende Wasser aus der Reaktionszone entweichen kann, um so eine Desaktivierung des Katalysators zu vermeiden.

In Soviet Inventions Illustrated, SU-707 904 (augegeben am 1. Oktober 1980) und in Soviet Invention Illustrated, SU-706 391 (ausgegeben am 24. September 1980) wird ein Verfahren zur Herstellung von Butylchloride beschrieben, das dadurch gekennzeichnet ist, daß Butanol mit Chlorowasserstoff in Flüssigphase in Gegenwart von Triethylamin-hydrochloride umgesetzt wird.

Aufgabe der vorliegenden Erfindung war es, ein Verfahren bereitzustellen, das es erlaubt, Methylhalogenide auf relativ einfache Weise und mit einer hohen Raum-Zeit-Ausbeute herzustellen, wobei die Bildung von Nebenprodukt auf ein Minimum reduziert werden soll. Diese Aufgabe wird durch die Erfindung gelöst.

Gegenstand der Erfindung ist ein Verfahren zur Herstellung von Methylhalogeniden, dadurch gekennzeichnet, daß Methanol mit Halogenwasserstoff in Flüssigphase in Gegenwart von Aminhydrohalogenid umgesetzt wird, wobei die Konzentration an freiem Halogenwasserstoff in der Flüssigphase unter der jeweiligen Azeotropkonzentration liegt.

Bei dem erfindungsgemäßen Verfahren werden als Aminhydrohalogenid vorzugsweise Hydrochloride und Hydrobromide, besonders bevorzugt Hydrochloride, von Aminen eingesetzt, wobei es sich bei den Aminen beispielsweise um primäre, sekundäre, tertiäre, lineare, cyclische, aliphatische und aromatische Amine handeln kann, mit der Maßgabe, daß die erfindungsgemäß eingesetzten Aminhydrohalogenide eine ausreichende thermische Stabilität aufweisen und in der flüssigen Phase, jeweils bezogen auf eine Temperatur von 25 °C, vorzugsweise zum Teil, besonders bevorzugt vollständig, löslich sind.

Beispiele für Amine im erfindungsgemäß eingesetzten Aminhydrohalogenid sind Ammoniak, Methylamin, Trimethylamin, Diethylamin, Triethylamin, n-Butylamin, Tributylamin, Ethylendiamin, 1,4-Diazabicyclo(2.2.2)octan, 3-Dimethylaminopropylamin, Diethylentriamin, Anilin sowie mit Halogenatomen und/oder Alkylgruppen substituierte Aniline, wie N,N-Dimethylanilin, o,m,p-Phenylendiamin, Heterocyclen, wie Chinoline, Imidazole, Piperidine und Piperazine, und Pyridin sowie substituierte Pyridine, wie mit Halogenatomen, Alkyl- und/oder Aminogruppen substituierte Pyridine.

Bevorzugte Amine im erfindungsgemäß eingesetzten Aminhydrohalogenid sind die aromatischen Amine, wie beispielsweise Aniline, Pyridine, Chinoline, Phenylendiamine sowie α- und β-Naphthylamin, wobei aromatische Amine mit niederem Molekulargewicht besonders bevorzugt sind.

Beispiele für die im erfindungsgemäßen Verfahren besonders bevorzugt eingesetzten Hydrohalogenide sind die Hydrochloride von Pyridin, 2-Methylpyridin, 4-Methylpyridin und Anilin.

Das im erfindungsgemäßen Verfahren eingesetzte Aminhydrohalogenid kann als solches, beispielsweise im Gemisch mit Wasser, in den Reaktor eingeführt werden oder dort aus dem entsprechenden Amin durch Umsetzung mit Halogenwasserstoff hergestellt werden.

Bei dem im erfindungsgemäßen Verfahren eingesetzten Aminhydrohalogenid kann es sich um eine einzelne Art wie auch um ein Gemisch aus mindestens zwei Arten derartiger Aminhydrohalogenide handeln.

Bei dem erfindungsgemäßen Verfahren enthält die Flüssigphase, bezogen auf das Gesamtgewicht der Flüssigphase, Aminhydrohalogenid in Mengen von vorzugsweise 10 bis 80 Gewichtsprozent, besonders bevorzugt 35 bis 60 Gewichtsprozent, jeweils berechnet als Gewicht des freien Amins.

Bei dem erfindungsgemäßen Verfahren wird Methanol in solchen Mengen eingesetzt, daß die Konzentration an Methanol in der Flüssigphase vorzugsweise zwischen 0,5 und 10 Gewichtsprozent, besonders bevorzugt zwischen 0,5 und 4 Gewichtsprozent, jeweils bezogen auf das Gesamtgewicht der Flüssigphase, liegt.

Bei dem im erfindungsgemäßen Verfahren eingesetzten Halogenwasserstoff handelt es sich vorzugsweise um Chlor- oder Bromwasserstoff, wobei Chlorwasserstoff besonders bevorzugt wird.

Besonders bevorzugt liegt die Konzentration an freiem Halogenwasserstoff in der Flüssigphase zwischen 0,1 und 19 Gewichtsprozent, insbesondere zwischen 0,1 und 10 Gewichtsprozent, jeweils bezogen auf das Gesamtgewicht der Flüssigphase. Als freier Halogenwasserstoff wird dabei der nicht an Amin gebundene Halogenwasserstoff bezeichnet.

Bei dem erfindungsgemäßen Verfahren wird die Umsetzung bei einer Temperatur von vorzugsweise 90 bis 200°C, besonders bevorzugt von 100 bis 160°C, und einem Druck von vorzugsweise 900 bis 16 000 hPa, besonders bevorzugt von 1 000 bis 6 000 hPa, durchgeführt, wobei die Reaktionsbedingungen vorzugsweise so gewählt werden, daß das Volumen der flüssigen Phase in etwa konstant bleibt.

Zur Durchführung des erfindungsgemäßen Verfahrens werden in einen Reaktor, der Aminhydrohalogenid im Gemisch mit Wasser und gegebenenfalls weiteren Stoffen enthält, Methanol und Halogenwasserstoff gegeben. Alkanol und Halogenwasserstoff können dabei jeweils sowohl in flüssigem als auch in gasförmigem Zustand, als gas/flüssig-Gemische oder in Form wäßriger Lösungen zugegeben werden. Das bei der Umsetzung entstehende Methyhalogenid wird in an sich bekannter Weise isoliert.

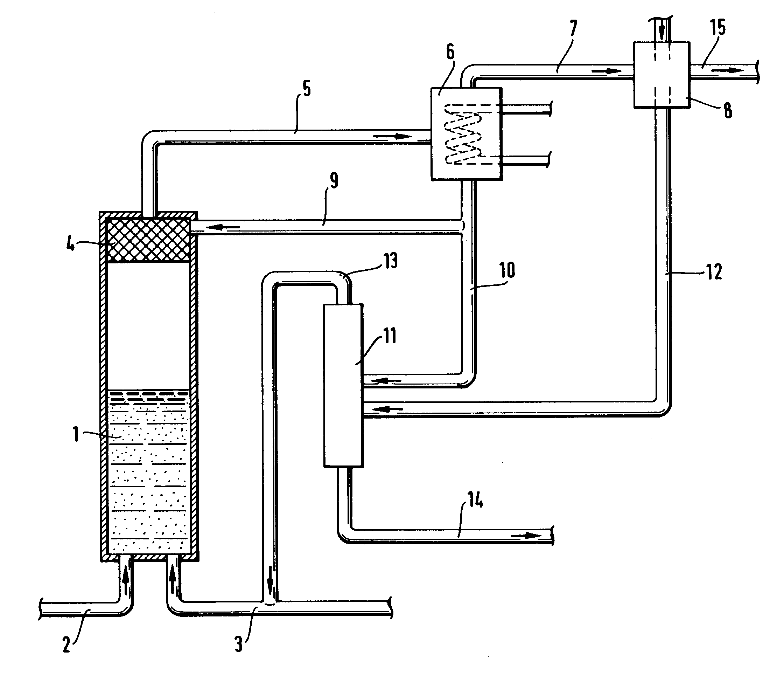

Eine bevorzugte Ausführungsform des erfindungsgemäßen Verfahrens soll anhand von Fig. 1 sowie Chlorwasserstoff und Methanol als Ausgangsstoffe erläutert werden.

In den heizbaren Reaktor (1), der Katalysator im Gemisch mit Wasser enthält, wird über Leitung (2) Chlorwasserstoff und über Leitung (3) Methanol zugespeist. Das bei der Umsetzung gebildete Chlormethan entweicht im Gemisch mit Wasser, Methanol und Spuren Chlorwasserstoff in den Gasraum und gelangt über Leitung (5) in den Kondensator (6), wo die Hauptmenge an Wasser und Methanol abgetrennt und über Leitung (10) der Destillationskolonne (11) zugeführt wird. Das am Kopf der Destillationskolonne (11) erhaltene Methanol wird über Leitung (13) in den Reaktor (1) eingespeist, während das im Sumpf der Destillationskolonne (11) enthaltene Wasser über Leitung (14) die Anlage verläßt. Aus dem Kondensator (6) wird das Chlormethan über Leitung (7) dem Wasserwäscher (8), einer Absorptionskolonne, in der das Chlormethan durch Kontaktierung mit Wasser von Methanol befreit wird, zugeführt und verläßt über Leitung (15) die Anlage. Das methanolhaltige Wasser aus dem Wasserwäscher (8) wird über Leitung (12) ebenfalls der Destillationskolonne (11) zugeführt.

Bei einer besonders bevorzugten Ausführung des erfindungsgemäßen Verfahrens befindet sich am Kopf des Reaktors (1) eine Füllkörperpackung (4), in die das Methanol und Wasser enthaltende Kondensat aus Kondensator (6) über Leitung (9) rückgeführt werden kann, wobei das den Reaktor (1) verlassende Gasgemisch mit dem rücklaufenden Kondensat kontaktiert wird. Das Verhältnis der Kondensatmenge, welche über Leitung (9) rückgeführt wird, zur Kondensatmenge, welche über Leitung (10) in die Destillationskolonne (11) gespeist wird, liegt dabei vorzugsweise zwischen 0 und 2, besonders bevorzugt zwischen 0 und 1.

Ein Vorteil des erfindungsgemäßen Verfahrens besteht darin, daß Methylhalogenid auf relativ einfache Weise und mit einer hohen Raum-Zeit-Ausbeute hergestellt werden können, wobei Nebenprodukte, wie etwa Dialkylether, nicht oder in einem nur sehr geringen Ausmaß gebildet werden. Des weiteren werden bei dem erfindungsgemäßen Verfahren hohe Reaktionsgeschwindigkeiten und dadurch bedlngt ein sehr geringer Austrag von Alkanol und insbesondere Halogenwasserstoff mit den Reaktionsprodukten erzielt.

In den nachstehend beschriebenen Beispielen beziehen sich alle Angaben über Teile und Prozentsätze, soweit nicht anders angegeben, auf das Gewicht. Als Rücklaufverhältnis wird im Folgenden das Verhältnis der Kondensatmenge, welche über Leitung (9) rückgeführt wird, zur Kondensatmenge, welche über Leitung (10) in die Destillationskolonne (11) gespeist wird, bezeichnet. Die Raum-Zeit-Ausbeute bezeichnet den auf die Volumeneinheit der Flüssigphase bezogenen Massenstrom des Produkts (kg/m³·h).

### Beispiel 1

Reaktor (1) besteht aus einem 1 500 mm langen Glasrohr mit einem Innendurchmesser von 50 mm, an das ein mit Quarzheizstäben betriebener Umlaufverdampfer angeflanscht ist. Der Kopf des Glasrohres ist mit einer 300 mm hohen Füllkörperschicht (BERL-Sättel aus Keramik, 6x6 mm) (4) gefüllt. In Reaktor (1) wird nun eine Lösung von 3-Dimethylaminopropylaminhydrochlorid in Wasser, hergestellt durch Einleiten von 622 g Chlorwasserstoff in eine Lösung von 1169 g Wasser und 557 g 3-Dimethylaminopropylamin, gegeben und bei einem Druck von 1 600 hPa zum Sieden gebracht.

Nach Beginn der Zuspeisung von Chlorwasserstoff über Leitung (2) und Methanol über Leitung (3) stellt sich ein Fließgleichgewicht ein, das wie folgt charakterisiert ist. Das Rücklaufverhältnis beträgt dabei 1,0.

| Zuspeisung: | |
|---|---|
| Chlorwasserstoff (2): | 461 g/h |
| Methanol (3): | 560 g/h |

| Flüssigphase: | |
|---|---|
| Volumen: | 2,2 l |
| Temperatur: | 118°C |
| Konzentration an freiem Chlorwasserstoff: | 9,2 % |
| Konzentration an Methanol: | 3,0 % |
| Konzentration des 3-Dimethylaminopropylaminhydrochlorids, berechnet als Gewicht des freien Amins: | 23,0 % |

| Austrag (5): | |
|---|---|
| Chlorwasserstoff: | 0,19 g/h |
| Methanol: | 156 g/h |
| Wasser: | 227 g/h |
| Chlormethan: | 132 g/h |
| Dimethylether: | 0,5 g/h |

Die Raum-Zeit-Ausbeute beträgt 290 kg/m³·h Chlormethan.

### Beispiel 2

In den in Beispiel 1 beschriebenen Reaktor (1) wird eine Lösung von Pyridinhydrochlorid in Wasser, hergestellt durch Einleiten von 588 g Chlorwasserstoff in eine Lösung von 816 g Wasser und 968 g Pyridin, gegeben und bei einem Druck von 1 600 hPa zum Sieden gebracht.

Nach Beginn der Zuspeisung von Chlorwasserstoff über Leitung (2) und Methanol über Leitung (3) stellt sich ein Fließgleichgewicht ein, das wie folgt charakterisiert ist. Das Rücklaufverhältnis beträgt dabei 0,3.

| Zuspeisung: | |
|---|---|
| Chlorwasserstoff (2): | 668 g/h |
| Methanol (3): | 800 g/h |

| Flüssigphase: | |
|---|---|
| Volumen: | 2,2 l |
| Temperatur: | 126°C |
| Konzentration an freiem Chlorwasserstoff: | 5,8 % |
| Konzentration an Methanol: | 2,0 % |
| Konzentration an Pyridinhydrochlorid, berechnet als Gewicht des freien Amins: | 40,0 % |

| Austrag (5): | |
|---|---|
| Chlorwasserstoff: | 0,36 g/h |
| Methanol: | 214 g/h |
| Wasser: | 329 g/h |
| Chlormethan: | 191 g/h |
| Dimethylether: | 0,2 g/h |

Die Raum-Zeit-Ausbeute beträgt 420 kg/m³·h Chlormethan.

### Beispiel 3

In den in Beispiel 1 beschriebenen Reaktor (1) wird eine Lösung von Pyridinhydrochlorid in Wasser, hergestellt durch Einleiten von 595 g Chlorwasserstoff in eine Lösung von 525 g Wasser und 1234 g Pyridin, gegeben und bei einem Druck von 1 600 hPa zum Sieden gebracht.

Nach Beginn der Zuspeisung von Chlorwasserstoff über Leitung (2) und Methanol über Leitung (3) stellt sich ein Fließgleichgewicht ein, das wie folgt charakterisiert ist. Das Rücklaufverhältnis beträgt dabei 1,0.

| Zuspeisung: | |
|---|---|
| Chlorwasserstoff (2): | 1280 g/h |
| Methanol (3): | 874 g/h |

| Flüssigphase: | |
|---|---|
| Volumen: | 2,2 l |
| Temperatur: | 125°C |
| Konzentration an freiem Chlorwasserstoff: | 1,1 % |
| Konzentration an Methanol: | 2,7 % |
| Konzentration an Pyridinhydrochlorid, berechnet als Gewicht des freien Amins: | 51,0 % |

| Austrag (5): | |
|---|---|
| Chlorwasserstoff: | 0,24 g/h |
| Methanol: | 513 g/h |
| Wasser: | 431 g/h |
| Chlormethan: | 250 g/h |
| Dimethylether: | 0,3 g/h |

Die Raum-Zeit-Ausbeute beträgt 550 kg/m³·h Chlormethan.

### Vergleichsbeispiel 1

In den in Beispiel 1 beschriebenen Reaktor (1) werden 3,1 l einer Lösung, bestehend aus 80 % Wasser, 16 % Chlorwasserstoff und 4 % Methanol, gegeben und bei einem Druck von 1 600 hPa zum Sieden gebracht.

Nach Beginn der Zuspeisung von Chlorwasserstoff über Leitung (2) und Methanol über Leitung (3) stellt sich ein Fließgleichgewicht ein, das wie folgt charakterisiert ist. Das Rücklaufverhältnis beträgt dabei 1,6.

| Zuspeisung: | |
|---|---|
| Chlorwasserstoff (2): | 291 g/h |
| Methanol (3): | 375 g/h |

| Flüssigphase: | |
|---|---|
| Volumen: | 3,1 l |
| Temperatur: | 116°C |
| Konzentration an freiem Chlorwasserstoff: | 16,6 % |
| Konzentration an Methanol: | 3,9 % |

| Austrag (5): | |
|---|---|
| Chlorwasserstoff: | 5 mg/h |
| Methanol: | 120 g/h |
| Wasser: | 144 g/h |
| Chlormethan: | 42 g/h |
| Dimethylether: | 0,3 g/h |

Die Raum-Zeit-Ausbeute beträgt 130 kg/m³·h Chlormethan.

## Patentansprüche

1. Verfahren zur Herstellung von Methylhalogeniden dadurch gekennzeichnet, daß Methanol mit Halogenwasserstoff in Flüssigphase in Gegenwart von Aminhydrohalogenid umgesetzt wird, wobei die Konzentration an freiem Halogenwasserstoff in der Flüssigphase unter der jeweiligen Azeotropkonzentration liegt.

2. Verfahren nach Anspruch 1, dadurch gekennzeichnet, daß als Halogenwasserstoff Chlorwasserstoff oder Bromwasserstoff eingesetzt wird.

3. Verfahren nach Anspruch 1 oder 2, dadurch gekennzeichnet, daß als Halogenwasserstoff Chlorwasserstoff eingesetzt wird.

4. Verfahren nach einem oder mehreren der Ansprüche 1 bis 3, dadurch gekennzeichnet, daß als Aminhydrohalogenid Hydrochloride von aromatischen Aminen eingesetzt werden.

5. Verfahren nach einem oder mehreren der Ansprüche 1 bis 4, dadurch gekennzeichnet, daß die Flüssigphase, bezogen auf das Gesamtgewicht der Flüssigphase, Aminhydrohalogenid in Mengen von 10 bis 80 Gewichtsprozent, berechnet als Gewicht des freien Amins, enthält.

6. Verfahren nach einem oder mehreren der Ansprüche 1 bis 5, dadurch gekennzeichnet, daß die Umsetzung bei einer Temperatur zwischen 90 und 200°C stattfindet.

7. Verfahren nach einem oder mehreren der Ansprüche 1 bis 6, dadurch gekennzeichnet, daß die Umsetzung bei einem Druck zwischen 1 000 und 16 000 hPa stattfindet.

## Claims

1. Process for the preparation of a methyl halide, characterized in that methanol is reacted with a hydrogen halide in the liquid phase in the presence of an amine hydrohalide, the concentration of free hydrogen halide in the liquid phase being below the particular azeotrope concentration.

2. Process according to Claim 1, characterized in that hydrogen chloride or hydrogen bromide is employed as the hydrogen halide.

3. Process according to Claim 1 or 2, characterized in that hydrogen chloride is employed as the hydrogen halide.

4. Process according to one or more of Claims 1 to 3, characterized in that a hydrochloride of an aromatic amine is employed as the amine hydrohalide.

5. Process according to one or more of Claims 1 to 4, characterized in that the liquid phase contains the amine hydrohalide in amounts of 10 to 80 percent by weight, based on the total weight of the liquid phase and calculated as the weight of free amine.

6. Process according to one or more of Claims 1 to 5, characterized in that the reaction takes place at a temperature between 90 and 200°C.

7. Process according to one or more of Claims 1 to 6, characterized in that the reaction takes place under a pressure between 1,000 and 16,000 hPa.

## Revendications

1. Procédé pour préparer des halogénures de méthyle, caractérisé en ce qu'on fait réagir du méthanol avec un halogénure d'hydrogène en phase liquide en présence d'un halogénhydrate d'amine, la concentration de l'halogénure d'hydrogène libre dans la phase liquide étant inférieure à la concentration correspondante de l'azéotrope.

2. Procédé selon la revendication 1, caractérisé en ce qu'on utilise comme halogénure d'hydrogène du chlorure d'hydrogène ou du bromure d'hydrogène.

3. Procédé selon la revendication 1 ou 2, caractérisé en ce qu'on utilise comme halogénure d'hydrogène du chlorure d'hydrogène.

4. Procédé selon l'une ou plusieurs des revendications 1 à 3, caractérisé en ce qu'on utilise comme halogénhydrate d'amine des chlorhydrates d'amines aromatiques.

5. Procédé selon l'une ou plusieurs des revendications 1 à 4, caractérisé en ce que la phase liquide contient, par rapport au poids total de la phase liquide, un halogénhydrate d'amine en des quantités de 10 à 80 % en poids, calculées en poids d'amine libre.

6. Procédé selon l'une ou plusieurs des revendications 1 à 5, caractérisé en ce que la réaction a lieu à une température comprise entre 90 et 200°C.

7. Procédé selon l'une ou plusieurs des revendications 1 à 6, caractérisé en ce que la réaction a lieu sous une pression comprise entre 1000 et 16 000 hPa.
